# EUROPEAN PATENT SPECIFICATION

(11) **EP 3 453 355 B1**
(45) Date of publication and mention of the grant of the patent: **26.06.2024**
(21) Application number: 17190193.7
(22) Date of filing: 08.09.2017
(51) Int. Cl.: A61B 34/20, A61B 90/00, A61B 17/02, A61B 1/313

(54) **SYSTEM FOR INHIBITING INJURY TO A PATIENT DURING LAPAROSCOPIC SURGERY**
SYSTEM ZUR HEMMUNG DER VERLETZUNG EINES PATIENTEN WÄHREND DER LAPAROSKOPISCHEN CHIRURGIE
SYSTÈME POUR L'INHIBITION DES LÉSIONS CHEZ UN PATIENT DURANT UNE CHIRURGIE LAPAROSCOPIQUE

(43) Date of publication of application: 13.03.2019
(73) Proprietor: Mariner Endosurgery Inc., Hamilton, ON L8R 2L2 (CA)
(72) Inventor: LANGLOIS, David Allen, Hamilton, ON L8K 5W4 (CA); SCHEMBRI, Lawrence, London, ON N6E 1Z5 (CA)
(74) Representative: Bobbert & Partner Patentanwälte PartmbB

(56) References cited:
- EP-A1- 2 612 606
- US-A1- 2015 238 276

## Description

### FIELD OF THE INVENTION

The present invention relates to systems for reducing accidental injury to patients during surgery and more particularly during laparoscopic surgery.

### BACKGROUND OF THE INVENTION

Compared with conventional surgery, laparoscopic surgery is an excellent means for achieving significant reductions in surgery-related morbidity. These reductions are achieved, however, only if the procedure is performed completely and without effective errors. Unfortunately, error-free laparoscopic surgeries are not the rule. Indeed, intra-operative and post-operative complications are all too common with laparoscopic surgery procedures. Because of this, there is a need to improve patient safety during laparoscopic surgery so that the benefits derived from such procedures are achieved while the drawbacks are reduced or eliminated.

One of the most profound drawbacks of laparoscopic surgery is the occurrence of unintentional or inadvertent injuries to patient tissue structures adjacent to or sometimes, distant from the intended surgical site or field. In the pelvic cavity, for example, bowels, ureters, large organs and blood vessels can be injured either directly from the heat or sharpness of the laparoscopic instruments, or burned indirectly through the conduction of heat through nearby tissues. Typically, such injuries are not appreciated at the time of surgery because the specific injury sites are hidden by blood or other patient tissues. As another disadvantage attendant to such iatrogenic injuries, the response to the unintended injury manifested by the patient is often a delayed one. This delayed response can be traumatic as well as tragic, and can sometimes result in one or more further surgeries, which would otherwise be unnecessary.

The implications from both a medical perspective as well as a medico-legal perspective are enormous. Obviously, such injuries are negative events and therefore best avoided. The present invention is therefore directed to reducing the occurrence and severity of these negative events.

EP 2 612 606 A1 relates to systems for reducing accidental injury to patients during surgery and more particularly during laparoscopic surgery. Safe zone position sensors are used to determine safe zones which help to position or move surgical instruments within a patient.

### SUMMARY OF THE INVENTION

The present invention is defined by the features of the independent claim. Embodiments of the invention are defined i.a. in the dependent claims.

In one aspect, there is provided a surgical system for use on a body of a patient, comprising a probe including an interior portion that is configured to be at least partially inserted into the body of the patient during use, a probe body to which the interior portion of the probe is connected, wherein the probe body is configured to be outside the body of the patient during use, wherein the interior portion of the probe includes a probing portion, and a probe marker on the probe body, a surgical instrument including an interior portion that is configured to be at least partially inserted into the body of the patient during use, an instrument body to which the interior portion of the surgical instrument is connected, wherein the instrument body is configured to be outside the body of the patient during use, wherein the interior portion of the surgical instrument includes a functional element, and an instrument marker on the instrument body, a laparoscope including an interior portion that is configured to be at least partially inserted into the body of the patient during use, a laparoscope body to which the interior portion of the laparoscope is connected, wherein the laparoscope body is configured to be outside the body of the patient during use, wherein the interior portion of the laparoscope includes an image receiving element, wherein, during use, the image receiving element is positionable in a surgical field in the body of the patient to receive images of the probing portion when the probing portion is in the surgical field and to receive images of the functional element when the functional element is in the surgical field, a display configured to display the images of the probing portion and of the functional element received by the laparoscope, a tracking system that includes at least one tracking system sensor positionable to track the probe marker during use of the probe and to track the instrument marker during use of the surgical instrument, a safe zone definition unit for determining safe zone positions without communicating with safe zone definition sensors optionally positioned on a netting - that may optionally be provided and positioned inside of the body of the patient, a controller, wherein the controller is programmed to (a) determine safe zone positions of the safe zone with the safe zone definition unit, the safe zone preferably being a zone within which the surgical instrument is allowed (e.g., as determined by the user or the procedure preceding the surgery or in advance to that) to be or within which it can operate without causing injury to the patient, (b) receive, e. g. continuous or substantially continuous, instrument marker input from the tracking system corresponding to a current position of the instrument marker which in turn corresponds to a current position of the functional element, (c) determine, e. g. continuously or substantially continuously, the current position of the functional element based on the instrument marker input, (d) determine, e. g. continuously or substantially continuously whether the functional element is within the safe zone based on the current position of the functional element determined in step (c), and (e) carry out at least one action if the controller determines in step (d) that the functional element is outside of the defined safe zone, wherein the at least one action is selected from the group of actions consisting of: notifying a user of the surgical instrument that the functional element is outside the safe zone; and disabling the functional element.

In another aspect of the disclosure not forming part of the invention, there is provided a method of using a surgical system on a body of a patient, comprising: (a) providing a probe including an interior portion that is configured to be at least partially inserted into the body of the patient during use, a probe body to which the interior portion of the probe is connected, wherein the probe body is configured to be outside the body of the patient during use, wherein the interior portion of the probe includes a probing portion, (b) providing a surgical instrument Including an interior portion that is configured to be at least partially inserted into the body of the patient during use, an instrument body to which the interior portion of the surgical instrument is connected, wherein the instrument body is configured to be outside the body of the patient during use, wherein the interior portion of the surgical instrument includes a functional element, (c) providing a laparoscope including an interior portion that is configured to be at least partially inserted into the body of the patient during use, a laparoscope body to which the interior portion of the laparoscope is connected, wherein the laparoscope body is configured to be outside the body of the patient during use, wherein the interior portion of the laparoscope includes an image receiving element, wherein, during use, the image receiving element is positionable in a surgical field in the body of the patient to receive images of the probing portion when the probing portion is in the surgical field and to receive images of the functional element when the functional element is in the surgical field, (d) defining, via a controller, a plurality of safe zone positions, wherein the controller is configured or programmed to detect when the probing portion is at a selected proximity to a periphery of the safe zone at each safe zone position without communicating with a netting - or preferably without communicating with safe zone definition sensors optionally positioned on the netting - that may optionally be provided and positioned inside of the body of the patient, wherein, for each safe zone position, the method further comprises: (e) receiving an indication from the controller when the probing portion is at the selected proximity to the periphery of the safe zone, (f) determining the position of the probe when an indication is received in step (e), (g) determining safe zone positions based on the position of the probe determined in step (f), wherein the method further comprises: (h) determining at least one safe zone position which defines the safe zone, the safe zone preferably being a zone within which the surgical instrument is allowed to be and/or in which it can operate without causing injury to the patient based at least in part on positions determined at step (g), (i) determining, e.g. several times, at intervals or substantially continuously, whether the functional element is within the safe zone, and (j) carrying out at least one action if the controller determines in step (i) that the functional element is outside of the safe zone, wherein the at least one action is selected from the group of actions consisting of: notifying a user of the surgical instrument that the functional element is outside the safe zone; and disabling the functional element.

In a further aspect, the disclosure defines or denominates a surgical field as a three-dimensional space in which the operative portions of laparoscopic instruments, those portions which are capable of causing harm to the patient or medical personnel, are permitted to function. In some embodiments, the hazardous or dangerous function of the instruments can be automatically attenuated or eliminated outside of this denominated space. The operative portions of a laparoscopic instrument or appliance include those that can potentially cause damage if they contact a patient's tissues in an unintended manner. Examples of such potentially damaging portions include hot wires, electrically charged wires, blades, scissors and shears, sharp points or surfaces. Thus, the operative portions can include those that are adapted and arranged to do one or more of cut, cauterize, ablate, seal, fuse, skewer or clamp.

In still another significant aspect, in order to track and monitor the relative positions and orientations of the instruments with respect to the protected space, and in order to track a probe used to assist in defining the protected space (i.e., a safe zone) the present disclosure employs one or more of software, optics, high speed digital imaging, such as visible spectrum or infrared (IR) imaging, 2D or 3D ultra sound, MRI and CAT scan images, visible spectrum or infrared (IR) imaging, photography, electromagnetic sensing, radio frequency (RF) sensing as well as one or more sensors to enable the surgeon, operating room and other medical personnel, including remote medical personnel, to be apprised of the precise positional status of the laparoscopic instruments being used.

Positional status refers to the relative position of the operative and non-operative portions of the various laparoscopic appliances and tools being used with respect to various portions of the patient's body, or with respect to the denominated surgical field, or with respect to one or more sensors placed inside or outside the patient's body.

A positive positional status refers to circumstances where the operative portions of the laparoscopic instruments are within the denominated boundaries of the surgical field. A neutral positional status refers to circumstances where the operative portions of the laparoscopic instruments are in the denominated surgical field but near at least one boundary. A negative positional status refers to circumstances where the operative portions of the laparoscopic instruments are outside of the denominated surgical field, or within a predetermined distance of a sensor.

Positional status is determined with respect to a three-dimensional surgical field having defined boundaries, or with respect to one or more sensors placed in proximity to vulnerable tissues. In accordance with certain aspects of the disclosure, those boundaries can be defined in a number of different ways and combinations thereof. For example, in some embodiments, proximity to one or more sensors placed on a vulnerable organ or tissue defines the boundaries of the protected space or denominated field. In other embodiments of the disclosure, the boundaries of the field can be determined with respect to distance from an object, such as a net used for sequestering the bowel, and the like. Thus, definition of the various boundaries makes it possible to determine the relative positions of various portions of laparoscopic instruments with respect to the denominated surgical field, and with respect to vulnerable tissues and organs, as well as with respect to various medical personnel.

Thus, in accordance with an embodiment of the disclosure, the three-dimensional spatial boundaries of a surgical field can be determined, or denominated, in a number of different ways. The present means and methods thus denominate the shape and volume of a three-dimensional space, and also track the position of portions of various instruments with respect to that space. By doing so, the likelihood of inadvertent damage is decreased. This is further enhanced by other aspects of the disclosure.

For example, each laparoscopic instrument being used in a particular procedure can have a range of statuses. Each of these statuses can be determined by the instrument's relative position in the denominated field, for example, by means of distance sensors, magnetic sensors, heat sensors, proximity sensors, 2D or 3D imaging technologies (ultra-sound, MRI, etc.) and the like.

Thus, a system of the present disclosure "knows" where inside of the body the operative portions of the laparoscopic instruments are located at all times. The sensors therefore aid the surgeon in staying away from vulnerable tissues and areas within the patient's body. Moreover, the instruments can be in operative communication, programmed or coded to shut off in the event that a dangerous structure is within the radius of a direct injury or a thermal burn, for instance. In an embodiment the invention reduces morbidity by providing the surgeon and other medical personnel with a "denominated surgical field" or "protected space" within which to perform the indicated procedure while reducing the risk of damage to other organs which, in essence, are provided with a kind of "force field" around them. Thus, in one aspect, the means and methods of the disclosure function to sequester vulnerable portions of the patient's body.

When the borders or limits of the denominated field are breached are approached, the system provides also for warnings to be given, such as a buzz or handle vibration in the laparoscopic tool being used. A system in accordance with an embodiment of the disclosure can thus be adapted and arranged such that the energized or sharp portions of the appliance are operational only within the boundaries determined by the sensor-enabled laparoscopic field, that is, the denominated field. As an example, in some embodiments, the means and methods of the disclosure can be adapted and arranged such that the sharp edges of the appliance are automatically withdrawn into one or more sheaths provided as part of the laparoscopic appliance.

In other embodiments of the disclosure, the means and methods of the disclosure can be effected by way of software that controls the various energy inputs to the laparoscopic instruments being used, thus preventing the unwanted cutting, avulsing, cauterizing, ablating, or severing of a patient's tissues and organs.

As yet another advantage, the means and methods of the present disclosure can also be adapted and arranged as teaching tools for providing virtually instantaneous feedback to surgeons and other medical personnel regarding their abilities and techniques in laparoscopic surgery. Various feedback loops and sensitivities of the disclosure can be adjusted to provide tailored instruction with respect to instructional or experimental surgeries on animals or models.

In some embodiments, all points, co-ordinates, positions and movements of instruments, body, organs and tissues can be recorded and stored for later playback if necessary. The playback can be provided in any of the following formats: audio, graphs, 2D graphic, and 3D graphic, or in any combination thereof.

In yet another aspect, the invention is directed to a surgical system for use on a body of a patient, wherein the system permits the user to determine the positions of a plurality of points on internal body portions of the patient surrounding a surgical field, wherein the points are used by a controller to determine a safe zone in which a functional element on a surgical instrument can be positioned without causing injury to the patient. The positions of the points may be monitored by the controller in real time so that if, after the safe zone is determined initially by the controller, the internal body portions of the patient move, the controller updates the data relating to the safe zone in real time. The system can employ a netting having magnetic or ferromagnetic elements and that is positioned in the surgical field to assist in determining the points that define the safe zone both initially and in real time during a medical procedure.

In still yet another aspect, the disclosure is directed to a method of using a surgical system on a body of a patient. The method is used to determine the positions of a plurality of points on internal body portions of the patient surrounding a surgical field, in order to determine a safe zone in which a functional element on a surgical instrument can be positioned without causing injury to the patient.

### BRIEF DESCRIPTION OF THE DRAWINGS

Figure 1 is a perspective view of a surgical system for use on the body of a patient in accordance with an embodiment of the present invention;
Figures 2a-2d illustrate the surgical system shown in Figure 1 being used to determine a safe zone within the patient in which a surgical instrument can be maneuvered without causing injury to the patient;
Figure 2e is a perspective view of a surgical instrument being used during a surgical procedure;
Figure 3 is a perspective view of an optional net that can be included with the system shown in Figure 1;
Figures 4a-4d are examples of markers that can be included on a probe shown in Figure 1 to permit tracking of the probe by a camera system;
Figure 5 is a magnified perspective view of the net shown in Figure 3
Figures 6a-6d are examples of markers that can be included on a surgical instrument shown in Figure 1 to permit tracking of the surgical instrument by a camera system; and
Figure 7 is an alternative probe for use with the system shown in Figure 1; and
Figures 8a and 8b are a flow diagram of the programming for a controller in the surgical system shown in Figure 1.

### DETAILED DESCRIPTION OF THE INVENTION

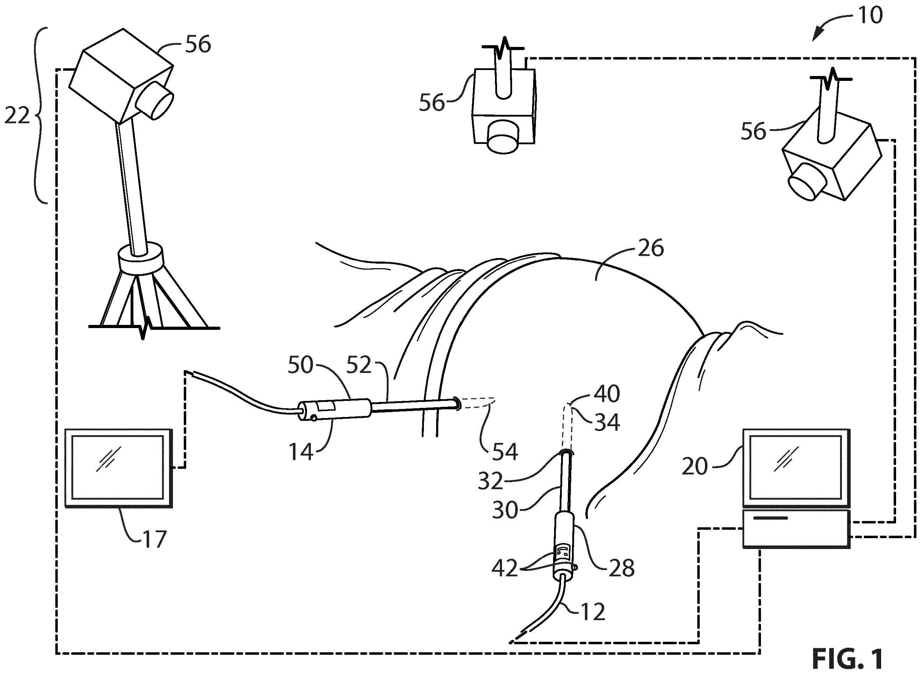

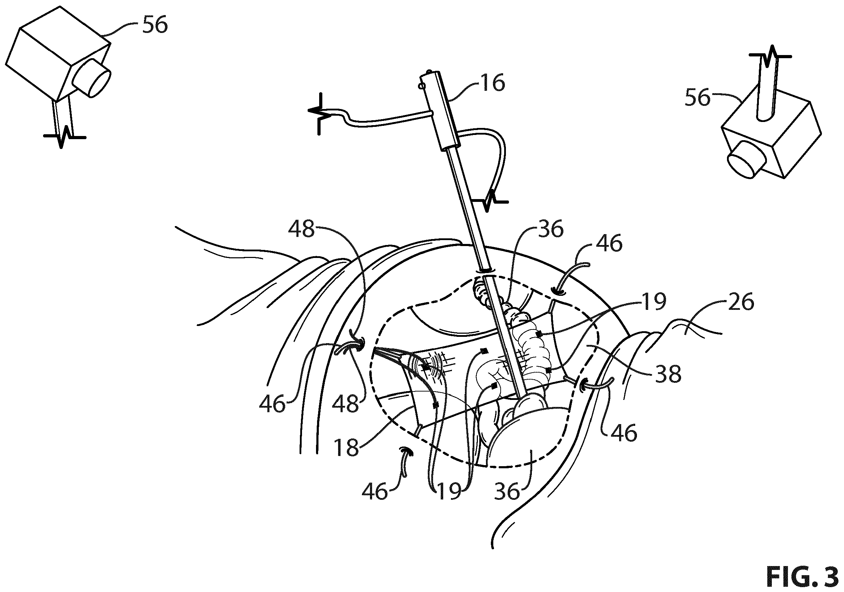

Reference is made to Figure 1 which shows a surgical system 10 for use on a body of a patient in accordance with an embodiment of the invention. The surgical system 10 includes a probe 12, a laparoscope 14, a surgical instrument 16 (Figure 3), a display 17, netting 18 (Figure 3) with a plurality of safe zone definition magnetic elements 19 on it, a controller 20 and a tracking system 22, which in the embodiment shown is a camera system. The surgical system 10 is configured to reduce the incidence of injuries to patients during laparoscopic surgery.

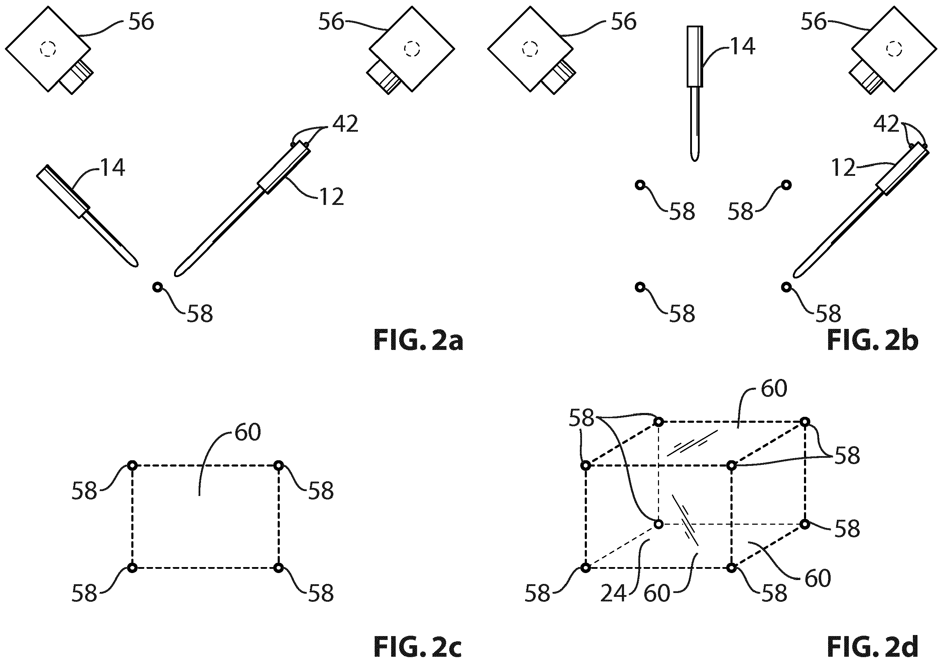

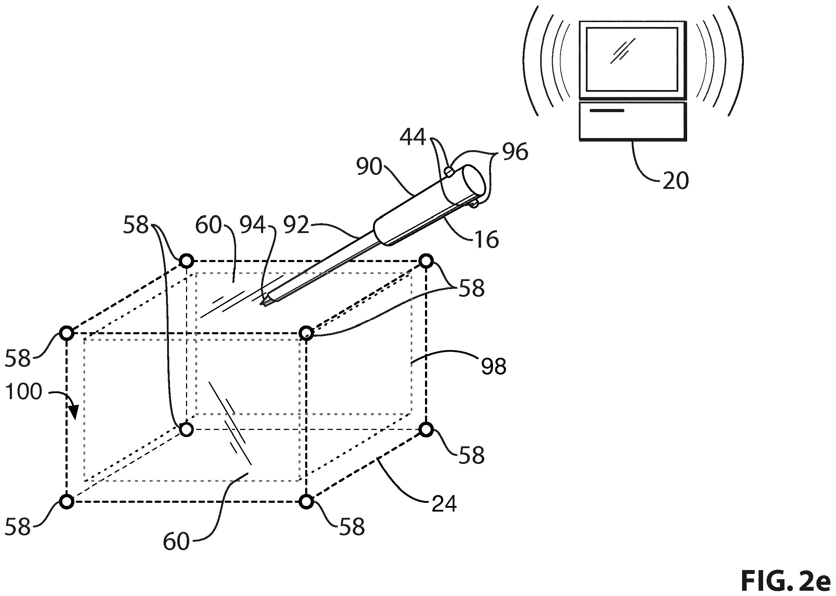

The system 10 is initially used to determine a safe zone 24 (Figure 2e) within the patient shown at 26 (only a portion of the patient 26 is shown in Figure 1) in which the surgical instrument 16 can be maneuvered without causing injury to the patient 26. The determination of the safe zone 24 involves the probe 12, the laparoscope 14 in particular. The probe 12 includes a probe body 28 and an interior portion 30 connected to the probe body 28. The interior portion 30 is configured to be at least partially inserted into the body of the patient 26 through one of a plurality of apertures 32 made in the body of the patient 26. The particular aperture 32 through which the probe 12 is inserted is shown at 32a. The interior portion 30 is therefore made from a material that will not cause harm to the patient, such as, for example, a suitable stainless steel. The probe body 28 is configured to be outside the body of the patient 26 during use.

The probe 12 further includes a probing portion 34 on the interior portion 30. The probing portion 34 is a portion of the interior portion 30 and is used to identify the positions of points on the internal body portions shown at 36 (Figure 3) of the patient 26 that are in the surgical field (i.e., that are in the vicinity of the particular site in the patient 26 that requires surgery). The surgical field is shown in Figure 3 at 38. Referring to Figure 2a, the probing portion 34 may be at a tip 40 of the interior portion 30.

A safe zone definition unit, discussed hereinbelow, includes at least one sensor at the probing portion 34 of the probe 12 for determining when the probing portion 34 is within or at the periphery of the safe zone 24.

During use of the probe 12, it is desired for the controller 20 to be able to determine the position of the probing portion 34 at selected times. To this end, a probe marker 42 is provided on the probe body 28. The probe marker 42 is, during use, viewed by the camera system 22 and is used by the controller 20 to identify the probe 12 (i.e., to distinguish the probe 12 over other objects, such as the instrument 16). Additionally or alternatively, the probe marker 42 is configured to provide sufficient information to the controller 20 for the controller 20 to be able to determine the position and orientation of the probe marker 34. By determining the position and orientation of the probe marker 34, the controller 20 can determine the position and orientation of the probe 12 itself and therefore can determine the position of the probing portion 34. Determining the position of the probing portion 34 is used by the controller 12 in determining where the internal body portions 36 of the patient 26 are, which is then used by the controller 20 to determine the safe zone 24.

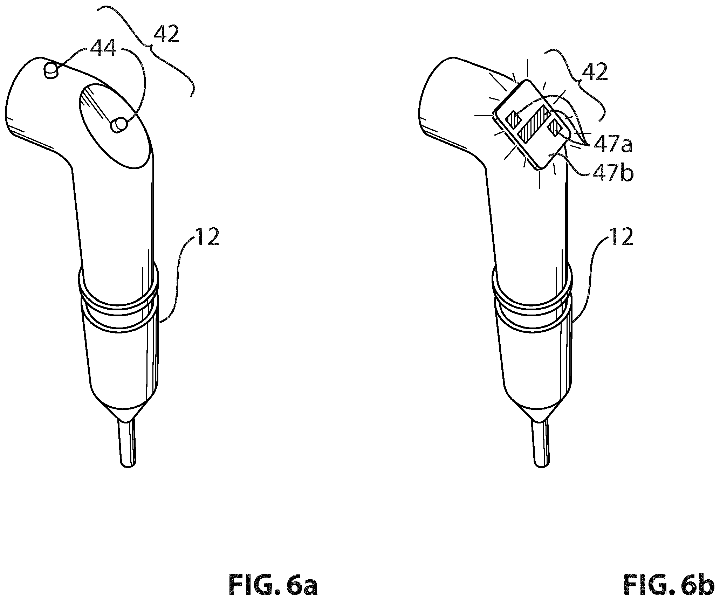

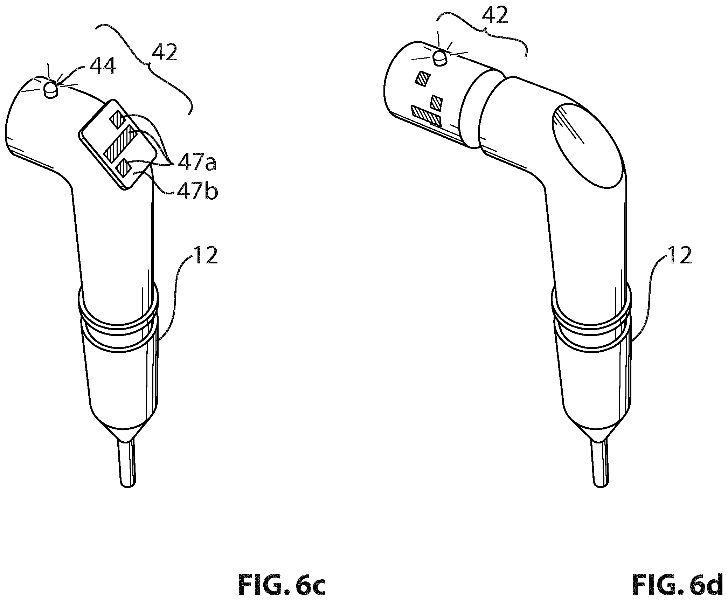

As shown in Figure 6a the probe marker 42 may, for example, be made up of a plurality of LEDs 44 on the probe 12. Some aspect of the LEDs 44 is unique so as to facilitate detection of the probe marker 42 in the images sent by the camera system 22 to the controller 20 and optionally to distinguish the probe marker 42 from an analogous marker on the surgical instrument 16. For example, the arrangement of the LEDs 44 on the probe body 28 may be distinguishable by the controller 20 to detect the probe marker 42 and optionally to identify it as the probe marker 42 as opposed to the aforementioned marker on the surgical instrument. Alternatively or additionally, the LEDs 44 may be configured to emit light at a particular wavelength or combination of wavelengths of light.

The LEDs 44 may be configured to emit light at a non-visible wavelength (e.g., infrared) so as to not distract the user of the probe 12 (e.g., the surgeon) during use.

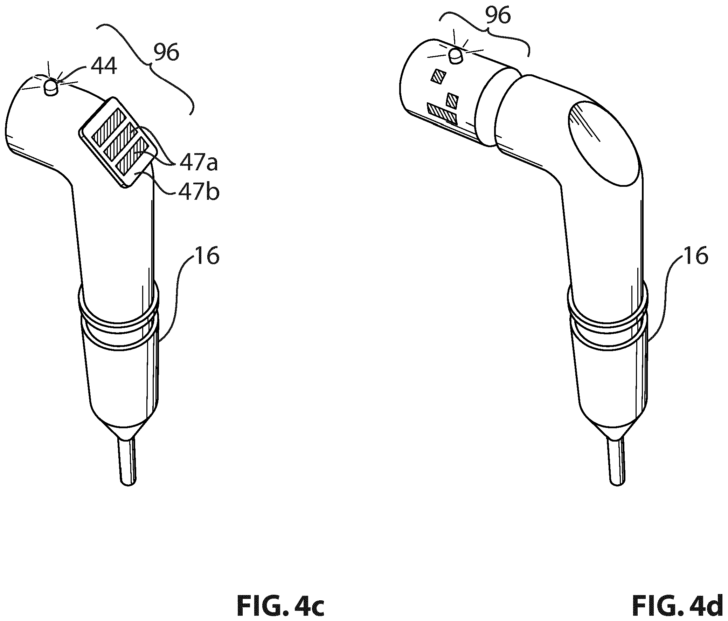

As an alternative to LEDs, the probe marker 42 may be made up of any suitable means for identifying the probe 12 and for identifying the position and orientation of the probe 12. For example, the probe marker 42 may include one or more symbols 47a (e.g., polygons) on a suitable background 47b as shown in Figure 6b. The colour of the symbols 47a and the colour of the background 47b may be selected to be of sufficient contrast to facilitate locating the symbols on the background, and may be selected to be sufficiently unique so as to permit the controller 20 to detect the probe marker 42 in the images provided by the camera system 22. Alternatively, as shown in Figure 6c, a combination of LEDs 44 and symbols 47a and a background 47b may be provided. As shown in Figure 4d, the probe marker 42 may be removable from the probe body 28. For example, the marker 42 may be provided on a sleeve.

The netting 18 may have several purposes. For example, the netting 18 may be positionable to restrain at least some of the internal body portions 36 in the surgical field 38 from obstructing the surgical instrument 16 when the surgical instrument 16 is being used in the surgical field 38. Alternatively, the netting 18 may simply be provided to conform to the shape of at least some of the internal body portions 36 in the surgical field 38. The netting 18 may be provided with any suitable means for restraining the internal body portions 36. For example, the netting 18 may be provided with a plurality of hold down members 46 which extend out of the body of the patient 26 and which may be attached to suitable attachment points on a support frame (not shown). Alternatively, the netting 18 may be provided with one or more hold down members that connect to other points within the body of the patient 26. Alternatively, the netting 18 may be provided with a grippy, elastic peripheral edge permitting the netting 18 to be mounted over internal body portions 36 and to hold on to the body portions 36 themselves. The netting 18 may be made up of one or more individual nets each of which is affixed to internal body portions 36 around the surgical field 38.

The probe 12 is configured to communicate with the controller 20 and to detect the magnetic elements 19 on the netting 18 to establish the positions of points on at least some internal body portions 36 in the surgical field 38 to assist in the determination of the safe zone 24 by the controller 20. The magnetic elements 19 may be any suitable type of magnet, such as, for example, a permanent magnet made of a rare-earth metal, or a ferromagnetic material, such as annealed iron.

The safe zone definition unit of the probe 12 is configured to detect when the probing portion 34 is at a selected proximity to the periphery of the safe zone 24, such as may be denoted by proximity to the netting 18.

In an exemplary embodiment, the safe zone definition unit may include one or more Hall Effect sensors that measure the magnetic force generated as a result of proximity to one or more of the magnetic elements 19. The controller 20 can determine the proximity to the magnetic elements 19 based on the magnetic force measured by the Hall Effect sensor(s).

In another embodiment, the netting may instead have a plurality of ferromagnetic elements, and the safe zone definition unit can include one or more magnetic elements in the probing portion that are biased towards a first position and actuatable towards a second position via a magnetic force between the magnetic elements and the ferromagnetic elements of the netting when the probing portion is at a selected proximity from the periphery of the safe zone 24. The location of the magnet between the first position and the second position provides an indication of the magnetic force and, thus, the proximity to one of the ferromagnetic elements on the netting.

In a further embodiment, the probing portion can include one or more light emitting elements, such as light emitting diodes ("LEDs"), lasers, etc. The light emitted can be within the spectrum range that is visible to a human, or may be outside of that range, such as infrared light. According to the invention the safe zone definition unit may include one or more light sensors in the probing portion for detecting the light emitted by the light emitting elements. For example, an infrared beam can be emitted by the light emitting element(s), and the light can be used to determine distance from a surface from which it is reflected that can be the periphery of the safe zone 24. The controller 20 can tell how far the light has travelled based on the phase shift of the received beam, as is well known in the art of proximity sensors.

Alternatively, the light emitting elements can project a grid or other reference pattern onto the tissue in front of the probe. The one or more light sensors can capture images of the reference pattern projected onto the tissue, and the reference pattern can be used to determine the proximity of the probe 12 to the periphery of the safe zone 24, such as via the transformation of the reference pattern.

The laparoscope 14 includes a laparoscope body 50 and an interior portion 52 connected to the laparoscope body 50. The interior portion 52 is configured to be at least partially inserted into the body of the patient 26 through one of the apertures 32. The particular aperture 32 through which the probe 12 is inserted is shown at 32b. The interior portion 52 is therefore made from a material that will not cause harm to the patient, such as, for example, a suitable stainless steel. The laparoscope body 50 is configured to be outside the body of the patient 26 during use.

The interior portion 52 includes an image receiving element 54. During use, the image receiving element 54 is positionable in the surgical field 38 in the body of the patient 26 to receive images of the probing portion 34 when the image receiving element 54 is in the surgical field 38. The image receiving element 54 may be a lens, for example. The laparoscope 14 is configured by any suitable means to transmit received images to the display 17. For example, the laparoscope 14 may include an image sensor (not shown), which may be, for example, a CCD sensor or a CMOS sensor, that is positioned to receive images from the image receiving element 54. The laparoscope 14 is configured to transmit the images of the probing portion 34 to the display 17 (optionally via a controller such as the controller 20).

The surgical instrument 16 includes an instrument body 90 and an interior portion 92 connected to the instrument body 90. The interior portion 92 is configured to be at least partially inserted into the body of the patient 26 during use. The instrument body is configured to be outside the body of the patient during use. The interior portion 92 includes a functional element 94, which is an element that is configured to perform a particular function on the patient. For example, the functional element 94 may be a cutting blade, a scissors mechanism or for example a heating element to cauterize. As will be understood, the functional element 94 may cause unintended injury to the patient 26 if it is accidentally brought into contact with the internal body portions 36 of the patient 26 surrounding the surgical field 38.

During use of the surgical instrument 16, it is desired for the controller 20 to be able to determine the position of the functional element 94 substantially continuously. To this end, an instrument marker 96 is provided on the instrument body 90. The instrument marker 96 is, during use, viewed by the camera system 22 and may be used by the controller 20 to identify the surgical instrument 16 (i.e., to distinguish the surgical instrument 16 over other objects, such as the probe 12). Additionally or alternatively, the instrument marker 96 is configured to provide sufficient information to the controller 20 for the controller 20 to be able to determine the position and orientation of the instrument 16. By determining the position and orientation of the instrument marker 96, the controller 20 can determine the position and orientation of the surgical instrument 16 itself and therefore can determine the position of the functional element 94. Determining the position of the functional element 94 is used by the controller 12 in determining whether the functional element 94 is within the safe zone 24.

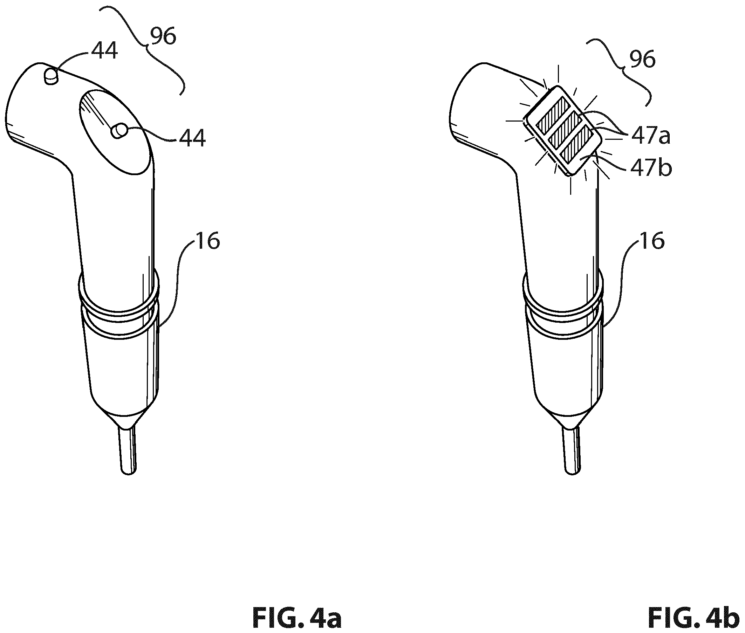

Some examples of instrument markers 96 are shown in Figures 4a, 4b, 4c and 4d. The instrument marker 96 may include LEDs 44 (Figure 4a), one or more symbols 47a (e.g., polygons) on a suitable background 47b (Figure 4b), or a combination of the two (Figure 4c). The instrument marker 96 may be removable from the instrument body 90 as shown in Figure 4d. For example, it may be provided on a sleeve.

The camera system 22 includes at least one camera 56 and preferably includes a plurality of cameras 56 mounted around the surgical theatre. The cameras 56 are positioned at selected positions to reduce the likelihood of obstruction of their view of the probe marker 42 and the instrument marker 96. The cameras 56 receive images of the probe marker 42 and transmit the images to the controller 20. The controller 20 is programmed to locate the probe marker 42 in the images and to determine by any suitable means, the position and orientation of the probe 12 and therefore the position of the probing portion 34. This may be achieved by comparing the images from two or more cameras 56 and using triangulation. Alternatively, a stereoscopic camera 56 may be used, so as to provide three-dimensional position information through images sent to the controller 20 without using multiple cameras. Alternatively, a single non-stereoscopic camera 56 may be used which sends a non-stereoscopic image to the controller 20. The controller 20 can determine easily the position of the marker 42 in the two-dimensional plane of the image easily and the depth of the probe marker 42 (i.e., its distance from the camera along a third dimensional axis perpendicular to the plane of the image) may be determined based on the apparent size of the marker 42 in the image.

Providing two or more cameras 56 may be advantageous to reduce the likelihood of the surgeon's hands or body from preventing the camera system 22 from obtaining an unobstructed view of the probe marker 42. In an embodiment where at least two cameras 56 are required to have an unobstructed view of the marker 42, the camera system 22 preferably includes 3 or more cameras 56.

Instead of incorporating cameras, the tracking system 22 could alternatively incorporate other types of tracking system sensor that is configured to sense the position of the probe marker and the instrument marker. For example, the tracking system could incorporate one or more of the following exemplary techniques to sense the position of the instrument 16 and of the probe 12: 2D or 3D ultra sound, MRI and CAT scan images, electromagnetic sensing, radio frequency (RF) sensing. Regardless of the technique used, and the technology used, whatever is on the probe and on the instrument that is detected by the tracking system may be considered a probe marker and an instrument marker respectively.

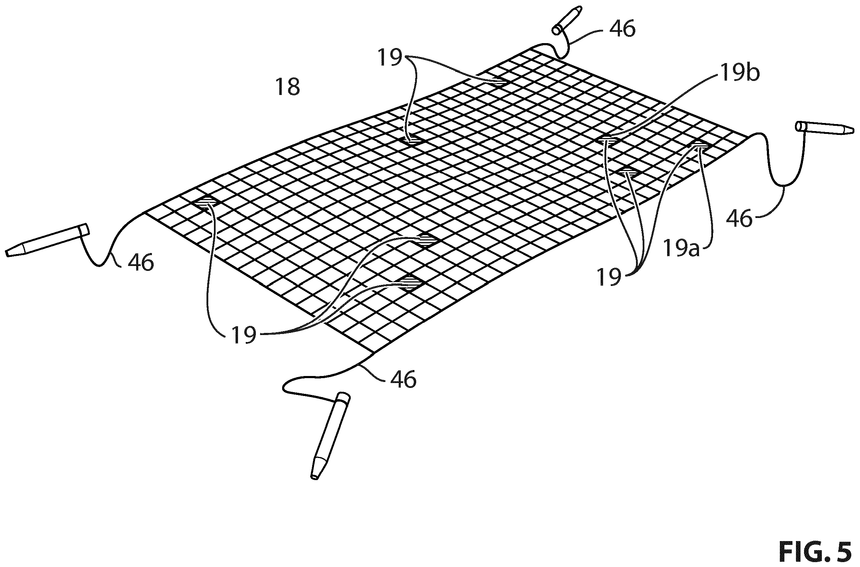

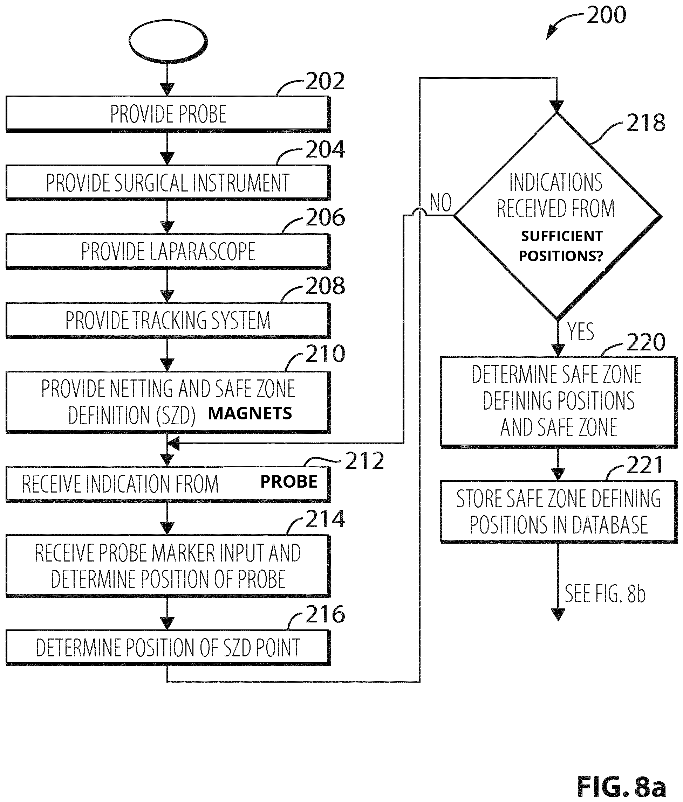

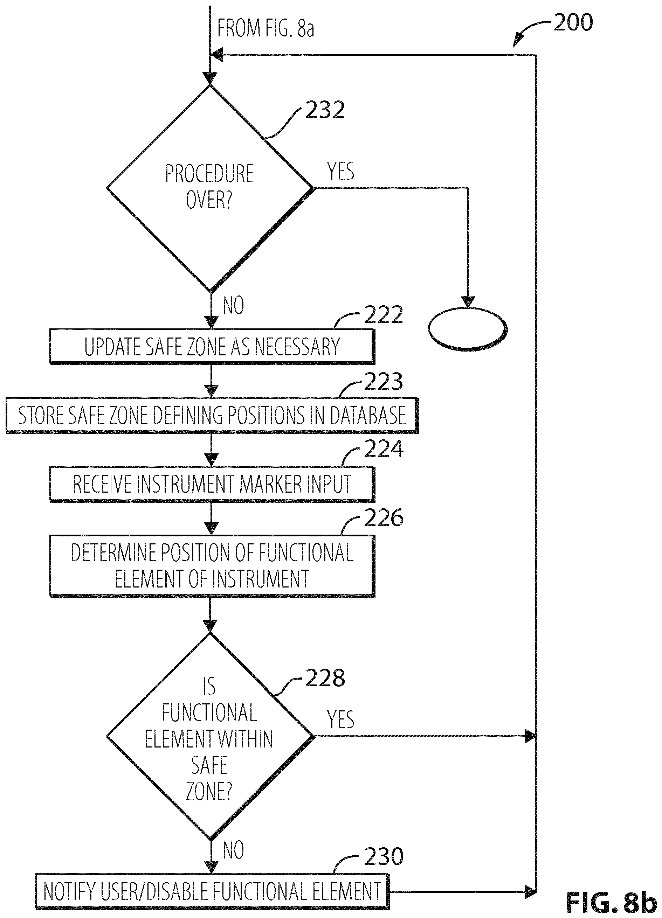

The operation of determining the safe zone 24 is as follows, with reference to Figures 1-6 and with reference to the flow diagram 200 shown in Figures 8a and 8b. Initially, a probe, a surgical instrument, a laparoscope, a tracking system, and netting with the magnetic elements 19 therein are provided in steps 202, 204, 206, 208 and 210 (Figure 8a). Then a plurality of points 58 on internal body portions 36 that surround the surgical field 38 are determined. To do this, the user creates the apertures 32. The user inserts the netting 18 with the magnetic elements 19 thereon into the surgical field 38 through one of the apertures 32 and affixes the netting 18 as desired. The user inserts the laparoscope 14 into the surgical field 38. The user inserts the probe 12 into the surgical field 38. The camera system 22 receives images of the probe marker 42 and transmits the images to the controller 20 (the images thus constitute probe marker input). The user can see the probing portion 34 of the probe 12 on the display 17 via the transmission of images from the laparoscope 14 to the display 17. Using the images from the laparoscope 14, the user guides the probe 12 so that the probing portion 34 contacts a first of the magnetic elements shown at 19a. When the probing portion 34 senses that it is within a selected proximity of the first magnetic element 19a, the safe zone definition unit in the probing portion 34 indicates the occurrence of the contact to the controller 20 (step 212). In this particular example, the first point 58a on the internal body portions 36 is substantially immediately adjacent the probing portion 34, since they are separated only by the magnetic element 19a, which may be thin. When the controller 20 receives the indication from the safe zone definition unit that contact was made, the controller 20 determines the position of the probing portion 34 of the probe 12 (step 214) based on the one or more images that were received from the camera system 22 at the time that the indication of contact from the magnetic element 19a was sent. The indication of the contact with the first magnetic element 19a, in combination with the one or more images from the camera system 22 may be considered input indicating the position of a first point 58a on the internal body portion 36. The controller 20 may use any suitable method for determining the position of the probing portion 34. The controller 20 uses the one or more images to determine the position and orientation of the probe marker 42, and thus the probe 12. The method used for this determination depends on whether the camera system 22 provides a single non-stereoscopic image, a plurality of non-stereoscopic images, or one or more stereoscopic images. It will be understood by one skilled in the art however, that many suitable algorithms exist for the determination of the position and orientation of an object using one or more images.

Once the controller 20 has determined the position and orientation of the probe 12, the controller 20 can then determine the position of the probing portion 34 based on the distance between a selected portion of the probe marker 42 and the probing portion 34 (which is a known value that is stored in the controller's memory). Using the position of the probing portion 34, the controller 20 can determine the position of the magnetic element 19a, and thus the position of point 58a (step 216). In this example, because the magnetic element 19a is substantially immediately adjacent the probing portion 34 and is thin, the determined position of the point 58a on the internal body portions 36 may be considered to be the same as the position of the probing portion 34. Once the position of the point 58a on the internal body portions 36 is determined, the controller 20 records it for use in determining the safe zone 24. After contacting the first magnetic element 19a, the user guides the probe 12 using the laparoscope 14 and display 17 so that the probing portion 34 contacts a second magnetic element 19b for the purpose of having the controller 20 determine the position of a second point 58b on the internal body portions 36. The user continues to guide the probe 12 from magnetic element 19 to magnetic element 19 until a satisfactory mapping of the safe zone 24 has been created using any one of a known set of techniques. In the flow diagram 200 this is shown by the controller 20 checking at step 218 if indications have been received from sufficient positions in the safe zone 24 to generate a definition of the periphery thereof and sending program control back to prior to step 212 if the answer to the check step 218 is 'no'.

While one particular magnetic element 19 was referred to in this example as the first magnetic element 19a, it will be understood that any of the magnetic elements 19 could have been referred to as the first magnetic element 19a, and any of the magnetic elements 19 could have been referred to as magnetic element 19b, and so on.

Once the positions of sufficient points 58 corresponding to the positions of magnetic elements 19 have been identified (i.e., the answer to check step 218 is 'yes') to define the safe zone 24, the controller 20 determines the safe zone 24 based on the points 58 (step 220). The points 58 may thus be referred to as safe zone defining points. The safe zone 24 may be determined by generating a plurality of virtual surfaces shown at 60 in Figure 2d between the points 58. The controller 20 may generate the virtual surfaces 60 between groups of points 58, as shown in Figure 2d. The surfaces 60 may, for example, be quadrilateral surfaces between groups of 4 points 58, or may be triangular surfaces between groups of 3 points 58, or may be surfaces having some other number of sides between correspondingly sized groups of points 58. The virtual surfaces 60 define the periphery of the safe zone 24, which can be considered to be a virtual conduit through which the functional element 94 of the instrument 16 can pass without causing injury to the patient 26.

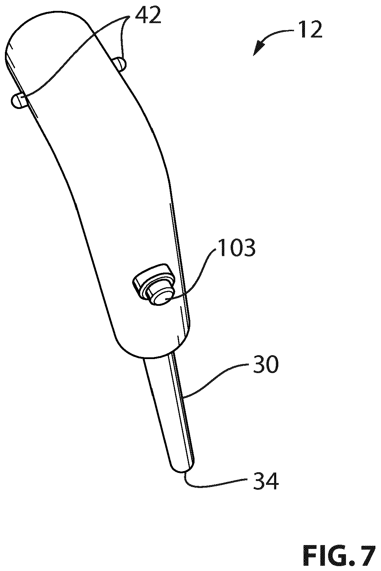

In addition to determining points 58 based on the positions of the magnetic elements 19, the probe 12 may be used to determine some points 58 that are not based on the magnetic elements 19. For example, the probe 12 may be positioned with the aid of the laparoscope 14 so that the probing portion 34 contacts the tip of a bone. When in contact with the bone, the user may indicate to the controller 20 to determine the position of the probing portion 34. For example, the probe 12 may include a button 103 as shown in Figure 7, which the user can press to indicate to the controller 20 to determine the position of the probing portion 34.

Once the points 58 have been determined, they may be stored in a database as shown at step 221. After the positions of the points 58 have been determined, the probe 12 may be removed from the patient 26.

In another embodiment, the netting 18 instead includes ferromagnetic elements, and the safe zone definition unit includes at least one actuatable magnetic element probing portion 34 that is biased towards a first position, such as via a coil spring or vacuum force, and can be actuated towards a second position by a magnetic force generated between the at least one magnetic element and the ferromagnetic elements of the netting 18 when in proximity thereof. The probing portion 34 can report to the controller 20 the location of the at least one magnetic element between the first position and the second position as a result of being within a selected proximity of one of the ferromagnetic elements of the netting 18.

In a further embodiment, a netting 18 may or may not be deployed at 210, and the safe zone definition unit projects a reference pattern via one or more light emitting elements in the probing portion 34 and captures images of the projected pattern on surrounding tissues or netting to determine the proximity of the probing portion 34 to the periphery of the safe zone 24 at a number of positions until sufficient data has been registered to define the safe zone 24.

The surgical instrument 16 is then used on the patient to carry out some task, such as cutting, cauterizing or some other suitable task. During use of the surgical instrument 16, it is possible that the internal organs of the patient may move. If the internal body portions 36 move during surgery it is important that the determined safe zone 24 be updated so as to continue to be useful in preventing inadvertent injury to the patient 26. In order to provide this capability, the positions defining the safe zone may be updated via use of the probe 12. This is represented as step 222 in Figure 8b. At step 223, the updated points are also stored in the database.

Any points 58 that were determined without the use of associated magnetic elements 19 can also be updated in a similar manner as before, or, alternatively, these points 58 may be considered by the controller 20 to be fixed (i.e., non-moving during the course of the medical procedure). Preferably any such points are points that are not expected to move during the medical procedure, such as points on certain bones.

During use of the surgical instrument 16, the camera system 22 receives images of the instrument marker 96 and sends the images (which may be referred to as instrument marker input) to the controller 20 (step 224). This can occur a number of times, at intervals, and/or substantially continuously. The controller 20 processes the input using a similar algorithm to that used for determining the position of the probing portion 34, to determine the position of the functional element 94 (step 226). This can occur a number of times, at intervals, and/or substantially continuously as the instrument marker input is received. This information is used to determine whether the functional element 94 is within the safe zone 24 (step 228). This can occur a number of times, at intervals, and/or substantially continuously as the position of the functional element is determined. If the functional element 94 is outside the safe zone 24 (i.e., the answer to check step 228 is 'no'), the controller 20 is programmed to carry out at least one action selected from the group of actions consisting of: notifying the user of the surgical instrument 16 that the functional element 94 is outside the safe zone 24; and disabling the functional element 94 (step 230).

Disabling the functional element 94 may be carried out in a number of ways depending on what makes up the functional element 94. For example, if the functional element 94 is a heating element, power may be cut to it. Alternatively, if the functional element 94 includes a sharp edge (e.g., a cutting blade), the instrument 16 may include a sheath, and may be configured to automatically cover the functional element 94 with the sheath.

The controller 20 may notify the user in any suitable way that the functional element 94 is outside the safe zone 24. For example, the controller 20 may be configured to generate a selected sound via a speaker, and/or may be configured to generate a selected image on the display 17.

If the functional element 94 is within the safe zone 24 (i.e., the answer at check step 228 is 'yes'), the controller 20 sends program control to step 232, wherein it checks if the medical procedure has been completed. This may be indicated by the user pressing a power button or some other control to let the system know to stop. If the procedure is over (i.e., the answer to check step 232 is 'yes'), then the program (and thus the method) ends. If the answer to the check step 232 is 'no', then the controller 20 continues to check and update the safe zone 24 as mentioned above at step 222 and to continue to receive instrument marker input at step 224.

The controller 20 may be programmed to divide the safe zone 24 (Figure 2e) into two or more sub-zones. For example, the safe zone 24 may be divided into a safest zone 98 and a danger zone 100. The safest zone 98 is a central portion of the safe zone 24. If the functional element 94 is kept within the safest zone 98 there is a reduced risk that the user will accidentally move the instrument 16 in such a way as to cause the functional element 94 to contact and cause injury to an internal body portion 36. The danger zone 100 is a peripheral portion of the safe zone 24. In other words, it is the portion of the safe zone 24 immediately inwardly adjacent to the virtual surfaces 60 that define the periphery of the safe zone 24. With the safe zone 24 thus divided into multiple sub-zones, the controller 20 may be configured to notify the user via sound and/or images on the display 17 whether the functional element 94 is in a relatively safer part of the safe zone 24 (e.g., the safest zone 98) or is in a relatively less safe part of the safe zone 24 (e.g., the danger zone 100). For example, a green bar may be displayed on the display 17 when the functional element 94 is within the safest zone 98, a yellow bar may be displayed on the display 17 when the functional element 94 is within the danger zone 100, and a red bar may be displayed when the functional element 94 is outside of the safe zone 24. In another embodiment, the controller 20 may be programmed to give the user a continuously changing indication of the distance of the functional element 94 from the periphery of the safe zone 24, via sound and/or images. For example, the controller 20 may be programmed to emit sound elements (e.g., beeps) at a selected frequency of emissions (e.g., 2 beeps per second) if the functional element 94 is relatively far from the periphery of the safe zone 24. If the functional element 94 moves closer to the periphery of the safe zone 24, the frequency of the beeps may gradually increase (e.g., up to, for example, five beeps per second). If the element 94 leaves the safe zone 24, the sound may become continuous.

After the surgical procedure is completed, the instrument 16, the laparoscope 14 and the netting 18 may be removed from the patient 26.

It will be understood that, while it is convenient to have the magnetic elements 19 on the netting 18, it is alternatively possible for at least some of the magnetic elements 19 to be provided without netting 18. These magnetic elements 19 could be inserted individually through an aperture 32 an applied directly to an internal body portion 36 using, for example, a mild adhesive. It will also be understood that the netting 18 may be provided without magnetic elements 19 on it. The netting 18 in such an instance can still be useful to assist in restraining internal body portions from obstructing the surgical instrument 16.

The controller 20 may be configured to record the movements of the surgical instrument and the data relating to the safe zone 24 (i.e., the positions of the safe zone defining points 58 throughout the medical procedure). The recording may be made a printed recording, or in a more preferred embodiment, the recording may be made as data written to a database stored on a computer readable medium, such as a flash memory so that the surgical procedure can be played back and reviewed. Instead of a database, the data could be stored in some other computer readable format such as a data file containing a simple list. The capability to play back and review the movements of the instrument and the safe zone in a medical procedure can be useful to for a variety of purposes. For example, the procedure can be reviewed and explained to students in order to train them in the safe carrying out of such a procedure. Also, in the event that there is a complication during the recovery of the patient, the procedure can be reviewed to ensure that there was no injury that occurred that is the source of the complication.

The recording of the data and the movements of the instrument can be provided in any suitable format, such as, for example, audio, graphs, 2D graphic, and 3D graphic, or some combination thereof.

Throughout this disclosure, the components, such as the cameras, the laparoscope, the safe zone definition sensors and the probe have been shown and described as communicating with the controller via suitable electrical conduits such as wires. It will be understood that it is alternatively possible for any of these components to communicate with the controller via wireless means, such as a Bluetooth^{®} connection.

It has been disclosed that the instrument marker 96 and the probe marker 42 be used to identify the instrument 16 and the probe 12 to the controller 20, (i.e., to distinguish each from each other and from any other components sensed by the controller 20). However, an element that is separate from the marker 42 or 96 could alternatively be provided on the instrument 16 and the probe 12 respectively to identify each to the controller 20. For example, a unique RFID tag can be provided on each to identify each to the controller 20.

While the above description constitutes a plurality of embodiments of the present invention, it will be appreciated that the present invention is susceptible to further modification and change without departing from the fair meaning of the accompanying claims.

## Claims

1. A surgical system for use on a body of a patient, comprising:
- a probe including an interior portion that is configured to be at least partially inserted into the body of the patient during use, a probe body to which the interior portion of the probe is connected, wherein the probe body is configured to be outside the body of the patient during use, wherein the interior portion of the probe includes a probing portion, and a probe marker on the probe body;
- a surgical instrument including an interior portion that is configured to be at least partially inserted into the body of the patient during use, an instrument body to which the interior portion of the surgical instrument is connected, wherein the instrument body is configured to be outside the body of the patient during use, wherein the interior portion of the surgical instrument includes a functional element, and an instrument marker on the instrument body;
- a laparoscope including an interior portion that is configured to be at least partially inserted into the body of the patient during use, a laparoscope body to which the interior portion of the laparoscope is connected, wherein the laparoscope body is configured to be outside the body of the patient during use, wherein the interior portion of the laparoscope includes an image receiving element, wherein, during use, the image receiving element is positionable in a surgical field in the body of the patient to receive images of the probing portion when the probing portion is in the surgical field and to receive images of the functional element when the functional element is in the surgical field;
- a display configured to display the images of the probing portion and of the functional element received by the laparoscope;
- a tracking system that includes at least one tracking system sensor positionable to track the probe marker during use of the probe and to track the instrument marker during use of the surgical instrument;
- a safe zone definition unit for determining safe zone positions;
- a controller, wherein the controller is programmed to:
(a) determine safe zone positions of the safe zone with the safe zone definition unit, the safe zone preferably being a zone within which the surgical instrument is allowed to be and/or in which it can operate without causing injury to the patient;
(b) receive, e. g. several times, at intervals or substantially continuous(ly), instrument marker input from the tracking system corresponding to a current position of the instrument marker which in turn corresponds to a current position of the functional element;
(c) determine, e. g. several times, at intervals or substantially continuously, the current position of the functional element based on the instrument marker input;
(d) determine, e. g. several times, at intervals or substantially continuously, whether the functional element is within the safe zone based on the current position of the functional element determined in step (c), and
(e) carry out at least one action if the controller determines in step (d) that the functional element is outside of the defined safe zone, wherein the at least one action is selected from the group of actions consisting of: notifying a user of the surgical instrument that the functional element is outside the safe zone; and disabling the functional element;
**characterized in that** the safe zone definition unit defines the safe zones positions without communicating with safe zone definition sensors being optionally positioned on a netting;
and the safe zone definition unit includes at least one of at least one image sensor, at least one laser sensor, and at least one light sensor, in particular an infrared sensor, located on the probe.

2. A surgical system as claimed in claim 1, the controller is further programmed to:
(f) determine at least one of the safe zone positions based on the probe marker input;
(g) determine whether the functional element is within a danger zone, wherein the danger zone is a peripheral portion of the safe zone, and
(h) notify the user of the surgical instrument that the functional element is in the danger zone, if the functional element is determined to be in the danger zone in step (g).

3. A surgical system as claimed in any of the previous claims, wherein the tracking system includes at least one tracking system camera positioned to receive images of the probe marker when the probe is being used on the patient and wherein the controller is programmed to determine the position of the probe based on the images of the probe marker and positioned to receive images of the instrument marker when the instrument is being used on the patient and wherein the controller is programmed to determine the position of the instrument based on the images of the instrument marker.

4. A surgical system as claimed in any of the previous claims, wherein the instrument marker is removable from the rest of the instrument.

5. A surgical system as claimed in any of the previous claims, wherein the controller is programmed to emit and alter an audible sound as the functional element approaches a periphery of the safe zone so as to provide to the user of the surgical instrument an indication of the proximity of the functional element to the periphery of the safe zone.

6. A surgical system as claimed in any of the previous claims, wherein the tracking system includes at least one tracking system camera positioned to receive images of the probe marker and wherein the controller is programmed to determine the position of the probe based on the images of the probe marker.

7. A surgical system as claimed in any of the previous claims, with a netting that is insertable into the body of the patient and is positionable to restrain at least some of the internal body portions of the patient from obstructing the surgical instrument, the netting including a plurality of hold-down elements which are attachable to points outside of the body of the patient to hold the netting in such a way as to restrain at least some of the internal body portions of the patient from obstructing the surgical instruments, and wherein at least one safe zone position can be defined to be positioned adjacent the netting.

8. A surgical system as claimed in claim 1, further comprising at least one of an infrared projection unit and a laser projection unit located on the probe and configured to project a pattern.

## Patentansprüche

1. Ein chirurgisches System zur Verwendung am Körper eines Patienten, umfassend:
- Eine Sonde mit einem Innenbereich, der dazu konfiguriert ist, während der Verwendung zumindest teilweise in den Körper des Patienten eingeführt zu werden, einem Sondenkörper, an den der Innenbereich der Sonde angeschlossen ist, wobei der Sondenkörper so konfiguriert ist, dass er während der Verwendung außerhalb des Körpers des Patienten liegt, wobei der Innenbereich der Sonde einen Sondierungsteil umfasst, und einem Sondenmarker am Sondenkörper;
- Ein chirurgisches Instrument mit einem Innenbereich, der dazu konfiguriert ist, während der Verwendung zumindest teilweise in den Körper des Patienten eingeführt zu werden, einem Instrumentenkörper, an den der Innenbereich des chirurgischen Instruments angeschlossen ist, wobei der Instrumentenkörper so konfiguriert ist, dass er während der Verwendung außerhalb des Körpers des Patienten liegt, wobei der Innenbereich des chirurgischen Instruments ein Funktionselement umfasst, und einem Instrumentenmarker am Instrumentenkörper;
- Ein Laparoskop mit einem Innenbereich, der dazu konfiguriert ist, während der Verwendung zumindest teilweise in den Körper des Patienten eingeführt zu werden, einem Laparoskopkörper, an den der Innenbereich des Laparoskops angeschlossen ist, wobei der Laparoskopkörper so konfiguriert ist, dass der Laparoskopkörper während der Verwendung außerhalb des Körpers des Patienten liegt, wobei der Innenbereich des Laparoskops ein Bildempfangselement umfasst, wobei das Bildempfangselement während der Verwendung in einem chirurgischen Feld im Körper des Patienten positionierbar ist, um Bilder des Sondierungsteils zu empfangen, wenn der Sondierungsteil sich im chirurgischen Feld befindet, und Bilder des Funktionselements zu empfangen, wenn das Funktionselement sich im chirurgischen Feld befindet;
- Ein Display, das dazu konfiguriert ist, die vom Laparoskop empfangenen Bilder des Sondierungsteils und des Funktionselements anzuzeigen;
- Ein Trackingsystem, das mindestens einen Trackingsystem-Sensor umfasst, der positionierbar ist, um den Sondenmarker während der Verwendung der Sonde und den Instrumentenmarker während der Verwendung des chirurgischen Instruments zu verfolgen;
- Eine Einheit zur Definition von Sicherheitszonen zur Bestimmung von Sicherheitszonenpositionen;
- Ein Controller, der so programmiert ist zum:
(a) Bestimmen von Positionen der Sicherheitszone mit der Einheit zur Definition von Sicherheitszonen, wobei die Sicherheitszone vorzugsweise eine Zone ist, in der das chirurgische Instrument sich befinden darf und/oder operieren kann, ohne den Patienten zu verletzen;
(b) Empfangen, z.B. mehrmals, in Intervallen oder im Wesentlichen kontinuierlich, von Eingaben des Instrumentenmarkers des Trackingsystems, die einer aktuellen Position des Instrumentenmarkers entsprechen, die wiederum einer aktuellen Position des Funktionselements entspricht;
(c) Bestimmen, z.B. mehrmals, in Intervallen oder im Wesentlichen kontinuierlich, der aktuellen Position des Funktionselements basierend auf den Eingaben des Instrumentenmarkers;
(d) Bestimmen, z.B. mehrmals, in Intervallen oder im Wesentlichen kontinuierlich, ob sich das Funktionselement innerhalb der Sicherheitszone befindet, basierend auf der in Schritt (c) bestimmten aktuellen Position des Funktionselements;
(e) Ausführen mindestens einer Aktion, wenn der Controller in Schritt (d) bestimmt, dass sich das Funktionselement außerhalb der definierten Sicherheitszone befindet, wobei die mindestens eine Aktion aus einer Gruppe von Aktionen ausgewählt wird, die besteht aus: Benachrichtigung eines Benutzers des chirurgischen Instruments, dass sich das Funktionselement außerhalb der Sicherheitszone befindet; und Deaktivierung des Funktionselements;
**Dadurch gekennzeichnet, dass** die Einheit zur Definition der Sicherheitszone die Positionen der Sicherheitszonen bestimmt, ohne mit optional auf einem Netz positionierten Sensoren zur Definition der Sicherheitszone zu kommunizieren;
und dass die Einheit zur Definition der Sicherheitszone mindestens einen von mindestens einem Bildsensor, mindestens einem Lasersensor und mindestens einem Lichtsensor, insbesondere einem Infrarotsensor, die jeweils an der Sonde angeordnet sind, umfasst.

2. Ein chirurgisches System gemäß Anspruch 1, wobei der Controller weiter programmiert ist zum:
(f) Bestimmen mindestens einer der Positionen der Sicherheitszone basierend auf den Eingaben des Sondenmarkers;
(g) Bestimmen, ob sich das Funktionselement innerhalb einer Gefahrenzone befindet, wobei die Gefahrenzone ein peripherer Teil der Sicherheitszone ist, und
(h) Benachrichtigen des Benutzers des chirurgischen Instruments, dass sich das Funktionselement in der Gefahrenzone befindet, wenn in Schritt (g) bestimmt wurde, dass das Funktionselement sich in der Gefahrenzone befindet.

3. Ein chirurgisches System gemäß einem der vorhergehenden Ansprüche, wobei das Trackingsystem mindestens eine Trackingsystem-Kamera umfasst, die positioniert ist, um Bilder des Sondenmarkers zu empfangen, wenn die Sonde am Patienten verwendet wird, und wobei der Controller so programmiert ist, dass der Controller die Position der Sonde basierend auf den Bildern des Sondenmarkers bestimmt, und positioniert ist, um Bilder des Instrumentenmarkers zu empfangen, wenn das Instrument am Patienten verwendet wird, und wobei der Controller so programmiert ist, dass der Controller die Position des Instruments basierend auf den Bildern des Instrumentenmarkers bestimmt.

4. Ein chirurgisches System gemäß einem der vorhergehenden Ansprüche, wobei der Instrumentenmarker vom Rest des Instruments abnehmbar ist.

5. Ein chirurgisches System gemäß einem der vorhergehenden Ansprüche, wobei der Controller so programmiert ist, dass der Controller ein akustisches Signal ausgibt und ändert, wenn sich das Funktionselement dem Rand der Sicherheitszone nähert, um dem Benutzer des chirurgischen Instruments eine Anzeige der Nähe des Funktionselements zum Rand der Sicherheitszone zu geben.

6. Ein chirurgisches System gemäß einem der vorhergehenden Ansprüche, wobei das Trackingsystem mindestens eine Trackingsystem-Kamera umfasst, die positioniert ist, um Bilder des Sondenmarkers zu empfangen, und wobei der Controller so programmiert ist, dass er die Position der Sonde basierend auf den Bildern des Sondenmarkers bestimmt.

7. Ein chirurgisches System gemäß einem der vorhergehenden Ansprüche, mit einem Netz, das in den Körper des Patienten einführbar ist und positionierbar ist, um zumindest einige der inneren Körperabschnitte des Patienten daran zu hindern, das chirurgische Instrument zu behindern, wobei das Netz eine Vielzahl von Befestigungselementen umfasst, die an Punkten außerhalb des Körpers des Patienten befestigt werden können, um das Netz so zu halten, dass zumindest einige der inneren Körperabschnitte des Patienten daran gehindert werden, die chirurgischen Instrumente zu behindern, und wobei mindestens eine Sicherheitszonenposition definiert werden kann, um neben dem Netz positioniert zu werden.

8. Ein chirurgisches System gemäß Anspruch 1, weiterhin umfassend mindestens eine von einer Infrarotprojektionseinheit und einer Laserprojektionseinheit, die sich an der Sonde befindet und dazu konfiguriert ist, ein Muster zu projizieren.

## Revendications

1. Un système chirurgical destiné à être utilisé sur le corps d'un patient, comprenant:
- une sonde comportant une partie intérieure configurée pour être au moins partiellement insérée dans le corps du patient pendant l'utilisation, un corps de sonde auquel la partie intérieure de la sonde est reliée, le corps de sonde étant configuré pour être placé à l'extérieur du corps du patient pendant l'utilisation, la partie intérieure de la sonde comportant une partie de sondage ainsi qu'un marqueur de sonde sur le corps de sonde;
- un instrument chirurgical comportant une partie intérieure configurée pour être au moins partiellement insérée dans le corps du patient pendant l'utilisation, un corps d'instrument auquel la partie intérieure de l'instrument chirurgical est reliée, le corps d'instrument étant configuré pour être placé à l'extérieur du corps du patient pendant l'utilisation, la partie intérieure de l'instrument chirurgical comportant un élément fonctionnel ainsi qu'un marqueur d'instrument sur le corps d'instrument;
- un laparoscope comportant une partie intérieure configurée pour être au moins partiellement insérée dans le corps du patient pendant l'utilisation, un corps de laparoscope auquel la partie intérieure du laparoscope est reliée, le corps de laparoscope étant configuré pour être placé à l'extérieur du corps du patient pendant l'utilisation, la partie intérieure du laparoscope comportant un élément récepteur d'images, dans lequel, pendant l'utilisation, l'élément récepteur d'images peut être positionné dans un champ chirurgical situé dans le corps du patient afin de recevoir des images de la partie de sondage lorsque la partie de sondage se trouve dans le champ chirurgical et afin de recevoir des images de l'élément fonctionnel lorsque l'élément fonctionnel se trouve dans le champ chirurgical;
- un écran configuré de manière à afficher les images de la partie de sondage et de l'élément fonctionnel reçues par le laparoscope;
- un système de suivi comportant au moins un capteur de système de suivi positionnable de manière à suivre le marqueur de sonde pendant l'utilisation de la sonde ainsi qu'à suivre le marqueur d'instrument pendant l'utilisation de l'instrument chirurgical;
- une unité de définition de zone de sécurité pour déterminer des positions de zone de sécurité;
- un contrôleur, le contrôleur étant programmé pour:
(a) déterminer les positions d'une zone de sécurité à l'aide de l'unité de définition de zone de sécurité,
la zone de sécurité étant de préférence une zone dans laquelle l'instrument chirurgical est autorisé à se trouver et/ou dans laquelle il peut fonctionner sans causer de blessure au patient;
(b) recevoir, par exemple à plusieurs reprises, à intervalles ou de manière pratiquement continue, des entrées du marqueur d'instrument provenant du système de suivi correspondant à une position actuelle du marqueur d'instrument qui, à son tour, correspond à une position actuelle de l'élément fonctionnel;
(c) déterminer, par exemple à plusieurs reprises, à intervalles ou de manière pratiquement continue, la position actuelle de l'élément fonctionnel en fonction des entrées du marqueur d'instrument;
(d) déterminer, par exemple à plusieurs reprises, à intervalles ou de manière pratiquement continue, si l'élément fonctionnel se trouve dans la zone de sécurité en fonction de la position actuelle de l'élément fonctionnel déterminée à l'étape (c), et
(e) exécuter au moins une action si le contrôleur détermine à l'étape (d) que l'élément fonctionnel se trouve en dehors de la zone de sécurité définie, au moins une action étant sélectionnée parmi le groupe d'actions consistant à notifier à l'utilisateur de l'instrument chirurgical que l'élément fonctionnel se trouve en dehors de la zone de sécurité ainsi qu'à désactiver l'élément fonctionnel;
**caractérisé en ce que** l'unité de définition de la zone de sécurité définit les positions de la zone de sécurité sans communiquer avec les capteurs de définition de la zone de sécurité, lesquels sont éventuellement positionnés sur un filet;
et **en ce que** l'unité de définition de la zone de sécurité comporte au moins un des éléments suivants: au moins un capteur d'images, au moins un capteur laser, au moins un capteur de lumière, notamment un capteur infrarouge, situé sur la sonde.

2. Le système chirurgical selon la première revendication où le contrôleur est en outre programmé pour:
(f) déterminer au moins une des positions de la zone de sécurité en fonction des entrées du marqueur de sonde;
(g) déterminer si l'élément fonctionnel se trouve dans une zone de danger, la zone de danger étant une partie périphérique de la zone de sécurité, et
(h) notifier à l'utilisateur de l'instrument chirurgical que l'élément fonctionnel se trouve dans la zone de danger, s'il est déterminé à l'étape (g) que l'élément fonctionnel se trouve dans la zone de danger.

3. Le système chirurgical selon l'une quelconque des revendications précédentes, où le système de suivi comporte au moins une caméra de système de suivi positionnée de manière à recevoir des images du marqueur de sonde lorsque la sonde est utilisée sur le patient et où le contrôleur est programmé pour déterminer la position de la sonde en fonction des images du marqueur de sonde et positionnée de manière à recevoir des images du marqueur d'instrument lorsque l'instrument est utilisé sur le patient et où le contrôleur est programmé pour déterminer la position de l'instrument en fonction des images du marqueur d'instrument.

4. Le système chirurgical selon l'une quelconque des revendications précédentes, où le marqueur d'instrument est amovible par rapport au reste de l'instrument.

5. Le système chirurgical selon l'une quelconque des revendications précédentes, où le contrôleur est programmé pour émettre et modifier un son audible lorsque l'élément fonctionnel approche une périphérie de la zone de sécurité, afin de fournir à l'utilisateur de l'instrument chirurgical une indication de la proximité de l'élément fonctionnel par rapport à la périphérie de la zone de sécurité.

6. Le système chirurgical selon l'une quelconque des revendications précédentes, où le système de suivi comporte au moins une caméra de système de suivi positionnée de manière à recevoir des images du marqueur de sonde et où le contrôleur est programmé pour déterminer la position de la sonde en fonction des images du marqueur de sonde.

7. Le système chirurgical selon l'une quelconque des revendications précédentes, doté d'un filet qui peut être inséré dans le corps du patient et qui peut être positionné de manière à empêcher au moins certaines parties internes du corps du patient d'obstruer l'instrument chirurgical, le filet comportant une pluralité d'éléments de retenue pouvant être fixés à des points situés à l'extérieur du corps du patient afin de maintenir le filet de manière à empêcher au moins certaines parties internes du corps du patient d'obstruer les instruments chirurgicaux, et où au moins une position de zone de sécurité peut être définie de manière à être positionnée à côté du filet.

8. Le système chirurgical selon la première revendication, comprenant en outre au moins une unité de projection infrarouge ainsi qu'une unité de projection laser situées sur la sonde et configurées pour projeter un motif.
